(19) Europäisches Patentamt
European Patent Office
Office européen des brevets

(11) EP 4 768 516 A1

(12) **EUROPEAN PATENT APPLICATION**
published in accordance with Art. 153(4) EPC

(43) Date of publication:
**01.07.2026 Bulletin 2026/27**

(21) Application number: **24856411.4**

(22) Date of filing: **16.08.2024**

(51) International Patent Classification (IPC):
***C08G 18/76*** *(2006.01)* ***C07C 265/14*** *(2006.01)*
***C08G 18/38*** *(2006.01)* ***G02B 1/04*** *(2006.01)*
***G02C 7/00*** *(2006.01)*

(52) Cooperative Patent Classification (CPC):
**C07C 265/14; C08G 18/38; C08G 18/76; G02B 1/04; G02C 7/00**

(86) International application number:
**PCT/JP2024/029210**

(87) International publication number:
**WO 2025/041722 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.08.2023 JP 2023136644**

(71) Applicant: **Mitsui Chemicals, Inc.**
**Tokyo 104-0028 (JP)**

(72) Inventor: **NAKAI, Shinnosuke**
**Omuta-shi, Fukuoka 836-8610 (JP)**

(74) Representative: **Mewburn Ellis LLP**
**Aurora Building**
**Counterslip**
**Bristol BS1 6BX (GB)**

# (54) XYLYLENE DIISOCYANATE COMPOSITION, POLYMERIZABLE COMPOSITION, RESIN, MOLDED BODY, OPTICAL ELEMENT, AND LENS

(57) A xylylene diisocyanate composition includes a xylylene diisocyanate and a specific compound. The specific compound has a peak in a range of chemical shift values of 2.44 ppm to 2.46 ppm based on chloroform in deuterated chloroform when the xylylene diisocyanate composition is mixed with the deuterated chloroform and measured by $^{1}$H-NMR measurement.

EP 4 768 516 A1

## Description

TECHNICAL FIELD

The present invention relates to a xylylene diisocyanate composition, a polymerizable composition, a resin, a molded article, an optical element, and a lens.

BACKGROUND ART

Conventionally, it has been known to react a xylylene diisocyanate composition with a polythiol to produce a resin which can be used for an optical element such as a lens (see Patent Document 1 below).

Citation List

Patent Document

Patent Document 1: International Publication No. WO2018/190290

SUMMARY OF THE INVENTION

PROBLEM TO BE SOLVED BY THE INVENTION

In the production of a resin as described in Patent Document 1, further improvement in heat resistance may be required.

The present invention provides a xylylene diisocyanate composition and a polymerizable composition which allow for the production of a resin having excellent heat resistance, and a resin, a molded article, an optical element, and a lens which have excellent heat resistance.

MEANS FOR SOLVING THE PROBLEM

The present invention [1] includes a xylylene diisocyanate composition including: a xylylene diisocyanate; and a specific compound having a peak in a range of chemical shift values of 2.44 ppm to 2.46 ppm based on chloroform in deuterated chloroform when the xylylene diisocyanate composition is mixed with the deuterated chloroform and measured by $^1$H-NMR measurement.

The present invention [2] includes the xylylene diisocyanate composition described in the above-described [1], wherein a ratio of the specific compound is 100 ppm or more based on mass.

The present invention [3] includes the xylylene diisocyanate composition described in the above-described [1] or [2], wherein a ratio of the specific compound is 10000 ppm or less based on mass.

The present invention [4] includes the xylylene diisocyanate composition described in the above-described [1] or [2], wherein a ratio of the specific compound is 3000 ppm or less based on mass.

The present invention [5] includes the xylylene diisocyanate composition described in any one of the above-described [1] to [4], including: at least one of a urea compound represented by the following chemical formula (1) and a urea compound represented by the following chemical formula (2), as the specific compound.

Chemical formula (1):

[Chemical formula 1]

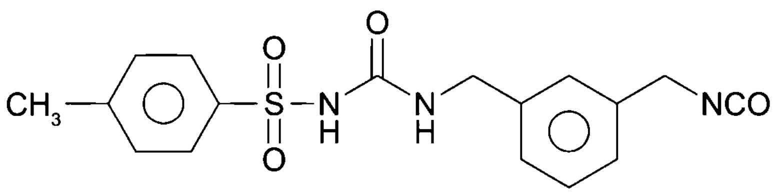

(1)

Chemical formula (2):

[Chemical formula 2]

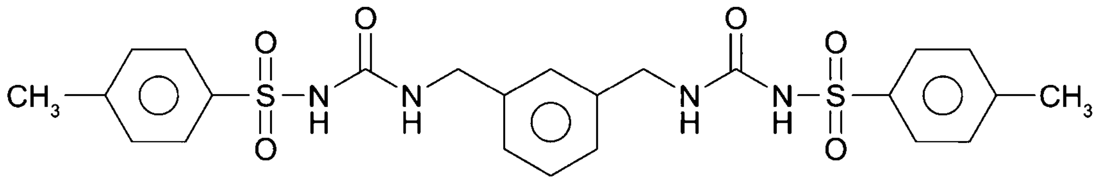
(2)

The present invention [6] includes the xylylene diisocyanate composition described in any one of the above-described [1] to [5], further including: at least one of 4-methylbenzenesulfonamide and 4-methylbenzenesulfonyl isocyanate.

The present invention [7] includes the xylylene diisocyanate composition described in the above-described [6], wherein a total sum of a ratio of the 4-methylbenzenesulfonamide in the xylylene diisocyanate composition and a ratio of the 4-methylbenzenesulfonyl isocyanate in the xylylene diisocyanate composition is 2000 ppm or less based on mass.

The present invention [8] includes the xylylene diisocyanate composition described in any one of the above-described [1] to [7], further including: at least one of monochloromethylbenzyl isocyanate and dichloromethylbenzyl isocyanate.

The present invention [9] includes the xylylene diisocyanate composition described in any one of the above-described [1] to [8], wherein the xylylene diisocyanate is obtained by reaction of xylylenediamine with carbonyl dichloride or by reaction of a hydrochloride salt of xylylenediamine with carbonyl dichloride.

The present invention [10] includes a polymerizable composition including: the xylylene diisocyanate composition described in any one of the above-described [1] to [9]; and an active hydrogen group-containing component.

The present invention [11] includes the polymerizable composition described in the above-described [10], wherein the active hydrogen-containing component contains at least one type of polythiol selected from the group consisting of 5,7-dimercaptomethyl-1,11-dimercapto-3,6,9-trithiaundecan, 4,7-dimercaptomethyl-1,11-dimercapto-3,6,9-trithiaundecan, 4,8-dimercaptomethyl-1,11-dimercapto-3,6,9-trithiaundecan, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, and ethylene glycol bis(3-mercaptopropionate).

The present invention [12] includes a resin being a cured product of the polymerizable composition described in the above-described [10] or [11].

The present invention [13] includes a molded article made of the resin described in the above-described [12].

The present invention [14] includes an optical element being the molded article described in the above-described [13].

The present invention [15] includes a lens being the optical element described in the above-described [14].

## EFFECTS OF THE INVENTION

The xylylene diisocyanate composition and polymerizable composition of the present invention contain a specific compound. The specific compound has a peak in a range of chemical shift values of 2.44 ppm to 2.46 ppm based on chloroform in deuterated chloroform when the xylylene diisocyanate composition is mixed with the deuterated chloroform and measured by $^1$H-NMR measurement.

By using the xylylene diisocyanate composition and polymerizable composition that contain the specific compound as a raw material, a resin having excellent heat resistance can be produced.

The resin of the present invention is a cured product of the polymerizable composition containing the above-described xylylene diisocyanate composition.

Further, the molded article, optical element, and lens of the present invention are made of the above-described resin.

Therefore, the resin, molded article, optical element, and lens of the present invention have excellent heat resistance.

## DESCRIPTION OF THE EMBODIMENTS

1. Xylylene Diisocyanate Composition

The xylylene diisocyanate composition (XDI composition) contains a xylylene diisocyanate (XDI) as a primary component. The "XDI composition" described here is a "second XDI composition" obtained by a "method of producing a XDI composition" described later.

Examples of the XDI include 1,2-XDI (o-XDI), 1,3-XDI (m-XDI), and 1,4-XDI (p-XDI.

As the XDI, preferably, 1,3-XDI(m-XDI) is used.

The XDI composition may contain two or more types of XDI.

The ratio (purity) of XDI in the XDI composition is, for example, 98.00% by mass or more, preferably 99.00% by mass or more, more preferably 99.30% by mass or more, still more preferably 99.60% by mass or more, and also, for example, 99.95% by mass or less.

The ratio of XDI in the XDI composition is measured by the method described in Examples described later.

The XDI composition contains a specific compound as a secondary component. The specific compound has a peak in a range of chemical shift values of 2.44 ppm to 2.46 ppm based on chloroform in deuterated chloroform when the xylylene diisocyanate composition is mixed with the deuterated chloroform and measured by $^1$H-NMR measurement.

$^1$H-NMR is measured under the measurement conditions described in Examples below.

The XDI composition contains at least one of a urea compound represented by the following chemical formula (1) and a urea compound represented by the following chemical formula (2), as the specific compound.

Chemical formula (1):

[Chemical formula 1]

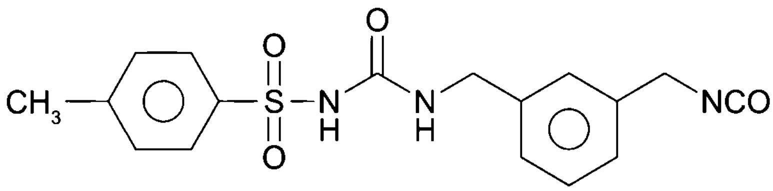

(1)

Chemical formula (2):

[Chemical formula 2]

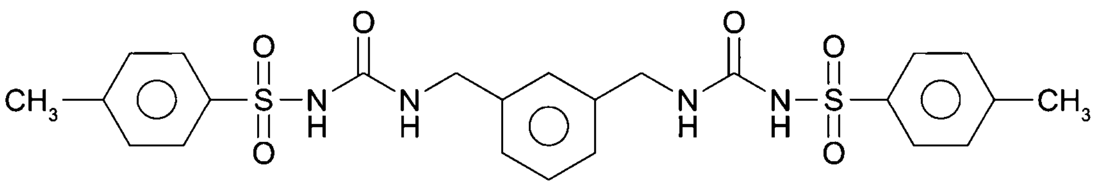

(2)

In addition, the XDI composition may contain a derivative obtained from XDI and 4-methylbenzene sulfonyl isocyanate (PTSI, also known as p-toluenesulfonyl isocyanate), as the specific compound. Examples of the derivative include an isocyanurate product, an allophanate product, a biuret product, a uretonimine product, a carbodiimide product, and a uretdione product.

When the specific compound is at least one of the urea compound represented by the above-described chemical formula (1) and the urea compound represented by the above-described chemical formula (2), the ratio of the specific compound in the XDI composition is, based on mass, for example, 1 ppm or more, preferably 10 ppm or more, more preferably 100 ppm or more, more preferably 300 ppm or more, more preferably 600 ppm or more, and more preferably 1000 ppm or more.

When the ratio of the specific compound in the XDI composition is the above-described lower limit or more, the

heat resistance of the resin produced using the XDI composition can be improved.

When the specific compound is at least one of the urea compound represented by the above-described formula (1) and the urea compound represented by the above-described formula (2), the ratio of the specific compound in the XDI composition is, based on mass, for example, 10000 ppm or less, preferably 7000 ppm or less, more preferably 5000 ppm, more preferably 3500 ppm or less, more preferably 3000 ppm, more preferably 2800 ppm or less, and more preferably, 2500 ppm or less.

When the ratio of the specific compound in the XDI composition is the above-described upper limit or less, the yellowness (YI value) of the resin produced using the XDI composition can be reduced.

Any one of the above-described upper limit values of the ratio of the specific compound in the XDI composition and any one of the above-described lower limit values of the ratio of the specific compound in the XDI composition can be combined to set a range of the ratio of the specific compound in the XDI composition.

The ratio of the specific compound in the XDI composition is measured by the method described in Examples described below.

The XDI composition may further contain, as a secondary component, at least one of PTSI and 4-methylbenzene sulfonamide (PTSA, also known as p-toluenesulfonamide).

The ratio of PTSI in the XDI composition is, based on mass, for example, 15000 ppm or less, 12000 ppm or less, more preferably 10000 ppm, more preferably 8000 ppm or less, more preferably 5000 ppm, more preferably 2000 ppm or less, more preferably 1000 ppm or less, more preferably 300 ppm or less, more preferably 100 ppm or less.

When the ratio of PTSI in the XDI composition is the above-described upper limit or less, the yellowness of the resin produced from the XDI composition can be reduced.

The ratio of PTSI in the XDI compositions is, based on mass, for example, more than 0 ppm, preferably 1 ppm or more, more preferably 5 ppm or more, more preferably 10 ppm or more, more preferably 20 ppm or more, more preferably 40 ppm or more.

When the ratio of PTSI in the XDI composition is the above-described lower limit or more, the storage stability of the XDI composition can be improved.

Any one of the above-described upper limit values of the ratio of PTSI in the XDI composition and any one of the above-described lower limit values of the ratio of PTSI in the XDI composition can be combined to set a range of the ratio of PTSI in the XDI composition.

The ratio of PTSI in the XDI composition is measured by the method described in Examples below.

The ratio of PTSA in the XDI composition is, based on mass, for example, 10000 ppm or less, more preferably 8000 ppm or less, more preferably 5000 ppm or less, more preferably 2000 ppm or less, more preferably 900 ppm or less, more preferably 500 ppm or less.

When the ratio of PTSA in the XDI composition is the above-described upper limit or less, it is possible to suppress an increase in the yellowness of the resin produced from the XDI composition due to the specific compound.

The ratio of PTSA in the XDI composition is, based on mass, for example, more than 0 ppm, preferably 1 ppm or more, more preferably 5 ppm or more, more preferably 10 ppm or more, more preferably 20 ppm or more, more preferably 40 ppm or more.

When the ratio of PTSA in the XDI composition is the above-described lower limit or more, the storage stability of the XDI composition can be improved.

Any one of the above-described upper limit values of the ratio of PTSA in the XDI composition and any one of the above-described lower limit values of the ratio of PTSA in the XDI composition can be combined to set a range of the ratio of PTSA in the XDI composition.

The ratio of PTSA in the XDI composition is measured by the method described in Examples below.

The total sum of the ratio of 4-methylbenzene sulfonamide in the xylylene diisocyanate composition and the ratio of 4-methylbenzene sulfonyl isocyanate in the xylylene diisocyanate composition is, based on mass, for example, 10000 ppm, preferably 7000 ppm or less, more preferably 5000 ppm or less, more preferably 2000 ppm or less, more preferably 1700 ppm or less, and more preferably 1500 ppm or less.

When the total sum of the ratio of 4-methylbenzene sulfonamide in the xylylene diisocyanate composition and the ratio of 4-methylbenzene sulfonyl isocyanate in the xylylene diisocyanate composition is the above-described upper limit or less, the yellowness of the resin produced from the XDI composition can be reduced, and the heat resistance of the resin produced using the XDI composition can be improved.

The total sum of the ratio of 4-methylbenzene sulfonamide in the xylylene diisocyanate composition and the ratio of 4-methylbenzene sulfonyl isocyanate in the xylylene diisocyanate composition is, based on mass, for example, more than 0 ppm, preferably 1 ppm or more, more preferably 5 ppm or more, more preferably 10 ppm or more, more preferably 20 ppm or more, more preferably 40 ppm or more, more preferably 100 ppm or more, more preferably 300 ppm or more, more preferably 500 ppm or more.

When the total sum of the ratio of 4-methylbenzene sulfonamide in the xylylene diisocyanate composition and the ratio of 4-methylbenzene sulfonyl isocyanate in the xylylene diisocyanate composition is the above-described lower limit or

more, the storage stability of the XDI composition can be improved.

The XDI composition may further contain, as a secondary component, at least one of monochloromethylbenzyl isocyanate (CBI) and dichloromethylbenzyl isocyanate (DCI).

The ratio of DCI in the XDI composition is, based on mass, for example, 0.1 ppm or more, preferably 0.3 ppm or more, more preferably 0.6 ppm or more, more preferably 1.0 ppm or more, and, for example, 60 ppm or less, preferably 50 ppm or less, more preferably 30 ppm or less, more preferably 20 ppm or less.

When the ratio of DCI in the XDI composition falls within the above-described range, it is possible to reduce the yellowness of the resin produced from the XDI composition.

The ratio of CBI in the XDI composition is, based on mass, for example, 0.2 ppm or more, preferably 6 ppm or more, more preferably 100 ppm or more, and, for example, 5000 ppm or less, preferably 4000 ppm or less, more preferably 3000 ppm or less, even more preferably 1600 ppm or less, particularly preferably 1000 ppm or less.

Further, the content ratio of CBI with respect to the content ratio of DCI is, for example, 2 times or more, preferably 10 times or more, more preferably 20 times or more, and, for example, 800 times or less, preferably 300 times or less, more preferably 50 times or less.

When the ratio of CBI in the XDI composition falls within the above-described range, it is possible to reduce the yellowness of the resin produced from the XDI composition. In particular, when the content ratio of CBI is the above-described upper limit or less, yellowing of the resin can be suppressed, and the urethanization reaction at the time of producing the resin can be smoothly progressed, and the mechanical properties of the resin can be reliably improved.

The ratio of DCI in the XDI composition and the ratio of CBI in the XDI composition are measured by the method described in Examples below.

2. Method of Producing XDI Composition

A method of producing the XDI composition is described.

The method of producing the XDI composition includes, for example, a synthesis step, a purification step, and a generation step.

In the synthesis step, XDI is synthesized. In the synthesis step, for example, XDI is synthesized by a hydrochloride method. When a hydrochloride method is employed, the synthesis step includes a salt-forming step and an isocyanate-forming step.

In the salt-forming step, xylylenediamine hydrochloric acid (XDA hydrochloride) is produced by mixing xylylenediamine (XDA) and hydrogen chloride.

Examples of XDA include 1,2-XDA (o-XDA), 1,3-XDA (m-XDA), and 1,4-XDA (p-XDA), and preferably 1,3-XDA (m-XDA) is used.

In the salt-forming step, for example, XDA and hydrogen chloride are reacted in the presence of an inert solvent. Specifically, hydrogen chloride gas is mixed with a solution in which XDA is dissolved in the inert solvent to react XDA with hydrogen chloride.

As the inert solvent, for example, the inert solvent described in paragraph [0059] of WO 2018/190290 is used. These inert solvents may be used alone or in combination of two or more. Among the inert solvents, preferably, halogenated aromatic hydrocarbons are used, and more preferably chlorobenzene and dichlorobenzene are used.

The mass ratio (total amine concentration) of XDA to the total sum of the mass of XDA and the inert solvent is, for example, 3% by mass or more, preferably 5% by mass or more, and, for example, 30% by mass or less, preferably 20% by mass or less, more preferably 15% by mass or less.

The feeding ratio of hydrogen chloride with respect to 1 mol of XDA is, for example, 2 mol or more, and, for example, 10 mol or less, preferably 6 mol or less, more preferably 4 mol or less.

The reaction temperature in the salt-forming step is, for example, 30°C or more, preferably 50°C or more, and, for example, 160°C or less, preferably 150°C or less, more preferably 140°C or less.

The reaction pressure (gauge pressure) in the salt-forming step is, for example, atmospheric pressure (0 MPaG) or more, preferably 0.01 MPaG or more, and, for example, 1.0 MPaG or less, preferably 0.5 MPaG or less.

The reaction of XDA with hydrogen chloride generates XDA hydrochloride, thereby producing a slurry that contains XDA hydrochloride.

Next, in the isocyanate-forming step, XDA hydrochloride and the carbonyl dichloride are reacted to produce a reaction mass which contains XDI. In the isocyanate-forming step, carbonyl dichloride is mixed into the slurry containing XDA hydrochloride to react XDA hydrochloride with carbonyl dichloride while removing the hydrogen chloride gas by-produced. The reaction of XDA hydrochloride with carbonyl dichloride generates XDI. That is, XDI is obtained by the reaction of XDA hydrochloride with carbonyl dichloride.

The feeding ratio of carbonyl dichloride with respect to 1 mol of XDA hydrochloride is, for example, 4 mol or more, preferably 5 mol or more, more preferably 6 mol or more, and, for example, 50 mol or less, preferably 40 mol or less, more preferably 30 mol or less.

The reaction time of the isocyanate-forming step is, for example, 4 hours or more, preferably 6 hours or more, and, for example, 25 hours or less, preferably 20 hours or less, more preferably 15 hours or less.

The reaction temperature in the isocyanate-forming step is, for example, 90°C or more, preferably 100°C or more, more preferably 110°C or more, and, for example, 190°C or less, preferably 180°C or less, more preferably 160°C or less.

The reaction pressure (gauge pressure) in the isocyanate-forming step is, for example, more than atmospheric pressure (0 MPaG), preferably 0.0005 MPaG or more, more preferably 0.001 MPaG or more, more preferably 0.003 MPaG or more, more preferably 0.01 MPaG (10 kPaG) or more, more preferably 0.02 MPaG (20 kPaG) or more, more preferably 0.03 MPaG (30 kPaG) or more, and, for example, 0.6 MPaG or less, preferably 0.4 MPaG or less, more preferably 0.2 MPaG or less.

The isocyanate-forming step is preferably carried out by a continuous process. In other words, the slurry containing XDA hydrochloride is continuously fed into the reaction tank used in the isocyanate-forming step, and the reaction mass is continuously taken out of the reaction tank while XDA hydrochloride and the carbonyl dichloride are being reacted in the reaction tank.

Next, the gas component, inert solvent, and tar component are removed from the reaction mass.

The gas component contains the carbonyl dichloride remaining in the reaction mass without reacting with XDA hydrochloride in the isocyanate-forming step, and the hydrogen chloride gas by-produced in the isocyanate-forming step. The gas component is removed from the reaction mass, for example, using a known degassing column.

The inert solvent is distilled off from the reaction mass, for example, using a known distillation column.

The tar component is removed from the reaction mass, for example, using a known tar removal device.

After the gas component, inert solvent, and tar component are removed, the ratio of XDI in the reaction mass is, for example, 80.0% by mass or more, preferably 90.0% by mass or more, more preferably 95.0% by mass or more, and, for example, 99.0% by mass or less, preferably 98.5% by mass or less, more preferably 98.0% by mass or less.

Next, in the purification step, the reaction mass is purified. The purification step includes, for example, a low-boiling removing step and a rectification step.

In the low-boiling removing step, a low boiling point component is removed from the reaction mass. The low boiling point component has a boiling point lower than that of XDI. In the low-boiling removing step, for example, the reaction mass is distilled in a low-boiling removal column to distill off the low boiling point component from the reaction mass.

Examples of the low-boiling removal column include a tray column and a packed column, and preferably, a packed column is used. The number of theoretical plates of the low-boiling removal column is, for example, 3 or more, preferably 5 or more, more preferably 7 or more, and, for example, 40 or less, preferably 20 or less, more preferably 15 or less.

The temperature of the bottom of the low-boiling removal column is, for example, 130°C or more, preferably 140°C or more, more preferably 150°C or more, and, for example, 200°C or less, preferably 190°C or less, more preferably 180°C or less.

The temperature of the top of the low-boiling removal column is, for example, 90°C or more, preferably 100°C or more, more preferably 110°C or more, and, for example, 160°C or less, preferably 150°C or less, more preferably 140°C or less.

The pressure of the top of the low-boiling removal column is, for example, 0.05 kPa or more, preferably 0.1 kPa or more, more preferably 0.2 kPa or more, and, for example, 3.0 kPa or less, preferably 2.0 kPa or less, more preferably 1.0 kPa or less.

The reflux ratio of the top of the low-boiling removal column is, for example, 1 or more, preferably 5 or more, more preferably 10 or more, and, for example, 80 or less, preferably 60 or less, more preferably 50 or less.

The residence time of the low-boiling removal column is, for example, 0.1 hours or more, preferably 0.2 hours or more, more preferably 0.3 hours or more, and, for example, 10 hours or less, preferably 5 hours or less, more preferably 3 hours or less.

By distillation using the low-boiling removal column, the reaction mass from which a low boiling point component is distilled off is obtained as a can effluent.

Next, in the rectification step, the reaction mass after the low-boiling removing step is further distilled (rectified) using a rectification column.

Examples of the rectification column include a tray column and a packed column, and preferably, a packed column is used. The number of theoretical plates of the rectification column is, for example, 1 or more, and, for example, 20 or less, preferably 10 or less, more preferably 5 or less.

The temperature of the bottom of the rectification column is 120°C or more, preferably 130°C or more, more preferably 140°C or more, and, for example, 190°C or less, preferably 180°C or less, more preferably 170°C or less.

The temperature of the top of the rectification column is, for example, 90°C or more, preferably 110°C or more, more preferably 130°C or more, and, for example, 180°C or less, preferably 170°C or less, more preferably 160°C or less.

The pressure of the top of the rectification column is, for example, 0.05 kPa or more, preferably 0.1 kPa or more, more preferably 0.2 kPa or more, and, for example, 3.0 kPa or less, preferably 2.0 kPa or less, more preferably 1.0 kPa or less.

The reflux ratio of the top of the rectification column is, for example, 0.1 or more, preferably 0.2 or more, more preferably 0.3 or more, and, for example, 50 or less, preferably 20 or less, more preferably 10 or less.

The residence time of the rectification column is, for example, 0.2 hours or more, preferably 0.5 hours or more, more preferably 1.0 hours or more, and, for example, 20 hours or less, preferably 10 hours or less.

By the rectification step, a fraction (first XDI composition) that contains XDI is produced. The first XDI composition contains DCI and CBI.

Next, in the generation step, the above-described urea compound is generated.

To produce the urea compound, for example, PTSA is added to the first XDI composition, and stirred while being heated.

The heating temperature in the generation step is, for example, 40°C or more, preferably 50°C or more, more preferably 60°C or more, and, for example, 120°C or less, preferably 100°C or less, more preferably 80°C or less.

The heating time in the generation step is, for example, 1 hours or more, preferably 3 hours or more, more preferably 5 hours or more, more preferably 8 hours or more, and, for example, 48 hours or less, preferably 36 hours or less, more preferably 24 hours or less, more preferably 12 hours or less.

By the generation step, PTSA and XDI are reacted to generate the above-described urea compound. Thus, the above-described XDI composition (second XDI composition) is obtained.

If necessary, PTSI may be added to the obtained XDI composition (second XDI composition) and mixed.

In addition, the method of producing the XDI composition does not include the generation step, and may include, for example, the salt-forming step, the isocyanate-forming step, the purification step, and the mixing step.

In the mixing step, the above-described urea compound and, if necessary, PTSA and PTSI are added to the first XDI composition, and mixed.

Also in this method, the above-described XDI composition can be obtained.

3. Uses of the XDI composition

The above-described XDI composition is utilized as a raw material for a resin. The resin is produced by reacting an isocyanate component containing the XDI composition with an active hydrogen group-containing component.

The active hydrogen group-containing component contains an active hydrogen group-containing compound.

Examples of the active hydrogen group-containing compound include a polyol, a polythiol, and a polyamine.

The active hydrogen group-containing compound can be used alone or in combination of two or more.

The active hydrogen group-containing compound is preferably a polythiol from the viewpoint of optical characteristics. The active hydrogen group-containing component is preferably a polythiol composition containing a polythiol as a primary component.

The ratio of polythiol in the polythiol composition is, for example, 50% by mass or more, preferably 60% by mass or more, more preferably 70% by mass or more, more preferably 80% by mass or more.

The ratio of polythiol in the polythiol composition is measured, for example, by high performance liquid chromatography.

The polythiol has a plurality of mercapto groups. The polythiol does not contain a secondary component to be described later. Examples of the polythiol include an aliphatic polythiol, an aromatic polythiol, and a heterocyclic polythiol.

Examples of the aliphatic polythiol include methanedithiol, 1,2-ethanedithiol, 1,2,3-propanetritiol, 1,2-cyclohexanedithiol, bis(2-mercaptoethyl)ether, tetrakis(mercaptomethyl)methane, diethylene glycol bis(2-mercaptoacetate), diethylene glycol bis(3-mercaptopropionate), ethylene glycol bis(2- mercaptoacetate), ethylene glycol bis(3-mercaptopropionate), trimethylolpropanetris(2-mercaptoacetate), trimethylolpropanetris(3-captopropionate), trimethylolethantris (2-mercaptoacetate), trimethylolethantris (3-mercaptopropionate), pentaerythritol tetrakis (2-mercaptoacetate), pentaerythritol tetrakis(3-mercaptopropionate), bis(mercaptomethyl)sulfide, bis(mercaptomethyl)disulfide, bis(mercaptoethyl) sulfide, bis(mercaptoethyl)disulfide, bis(mercaptopropyl)sulfide, bis(mercaptomethylthio)methane, bis(2-mercaptoethylthio)methane, bis(3-mercaptopropylthio)methane, 1,2-bis(mercaptomethylthio)ethane, 1,2-bis(2-mercaptoethylthio)ethane, 1,2-bis(3-mercaptopropylthio)ethane, 1,2,3-tris(mercaptomethylthio)propane, 1,2,3-tris(2-mercaptoethylthio)propane, 1,2,3-tris(3-mercaptopropylthio)propane, 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl) sulfide, 2,5-dimercaptomethyl-1,4-dithiane, 2,5-dimercapto-1,4-dithiane, 2,5-dimercaptomethyl-2,5-dimethyl-1,4-dithiane, and esters of thioglycolic acids and mercaptopropionic acids thereof, hydroxymethyl sulfide bis (2-mercaptoacetate), hydroxymethyl sulfide bis (3-mercaptopropionate), hydroxyethyl sulfide bis(2-mercaptoacetate), hydroxyethyl

sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), thiodiglycolic acid bis(2-mercaptoethyl ester), thiodipropionic acid bis(2-mercaptoethyl ester), dithiodiglycolic acid bis(2-mercaptoethyl ester), dithiodipropionic acid bis(2-mercaptoethyl ester), 1,1,3,3-tetrakis(mercaptomethylthio)propane, 1,1,2,2-tetrakis(mercaptomethylthio)ethane, 4,6-bis(mercaptomethylthio)-1,3-dithiane, 2-(2,2-bis(mercaptomethylthio)ethyl)-1,3-dithiethane, 3-mercaptomethyl-1,5-dimercapto-2,4-dithiapentane, tris(mercaptomethylthio)methane, and tris(mercaptoethylthio)methane.

Examples of the aromatic polythiol include 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(mercaptoethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptomethyleneoxy)benzene, 1,3,5-tris(mercaptoethyleneoxy)benzene, 2,5-toluenedithiol, 3,4-toluenedithiol, 1,5-naphthalenedithiol, and 2,6-naphthalenedithiol.

Examples of the heterocyclic polythiol include 2-methylamino-4,6-dithiol-sym-triazine, 3,4-thiophenedithiol, and bismuthiol.

The polythiols may be used alone or in combination of two or more.

Preferably, examples of the polythiol include at least one type selected from the group consisting of 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl)sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, ethylene glycol bis(3-mercaptopropionate), and diethylene glycol bis(3-mercaptopropionate).

The polythiol composition may contain a secondary component.

Examples of the secondary component include a compound in which at least one of a plurality of mercapto groups of the above-described polythiol is substituted with a functional group represented by the following chemical formula (3) (hereinafter, referred to as a compound A).

Chemical formula (3):

[Chemical formula 3]

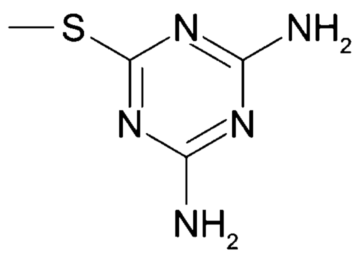

(3)

When the polythiol composition contains the compound A, in high performance liquid chromatography measurement of the polythiol composition, the peak area (R1) of the compound A with respect to the peak area 100 of the polythiol is, for example, 3.0 or less, preferably 1.5 or less, more preferably 0.50 or less, and, for example, 0.01 or more.

The "peak area (R1) of the compound A with respect to the peak area 100 of the polythiol" means the relative value (ratio) of the peak area ($P_A$) of the compound A when the peak area of the polythiol ($P_{thiol}$) (including structural isomers of the polythiol) ($P_{thiol}$) is 100, and is calculated according to the following formula (1).

$$\text{Formula (1): } R1 = (P_A/P_{thiol}) \times 100$$

When the "peak area (R1) of the compound A with respect to the peak area 100 of the polythiol" is determined, the high performance liquid chromatography measurement is carried out under the measurement conditions described in paragraph [0041] of WO2022/102625.

When the peak area (R1) of the compound A with respect to the peak area 100 of the polythiol is the above-described lower limit or more and the above-described upper limit or less, the pot life of the polymerizable composition obtained from the polythiol composition and the polyisocyanate composition can be maintained sufficiently, and further, by curing the polymerizable composition, a plastic lens made of a polythio urethane resin having excellent qualities, such as hue, transparency, striae, can be obtained.

In addition, when the polythiol composition contains at least one type of polythiol selected from the group consisting of 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, and 4,8-dimercaptomethyl-1,11-dimercapto-3,6,9-trithiaundecane, the polythiol composition may further contain a compound represented by the following chemical formula (4) (hereinafter, referred to as a compound B) as a secondary component.

Chemical formula (4):

[Chemical formula 4]

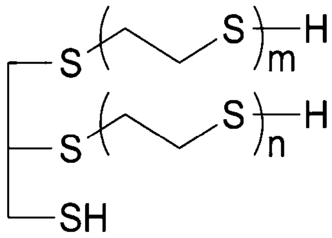

(4)

(In Formula (1), each of m and n independently represents 0 or 1, and m+n=1 is satisfied.)

When the polythiol composition contains the compound B, in the high performance liquid chromatography measurement of the polythiol composition, the peak area (R2) of the compound B with respect to the total peak area 100 of the compound contained in the polythiol composition is, for example, 10.0 or less, preferably 9.0 or less, more preferably 8.0 or less, more preferably 7.0 or less, more preferably 6.0 or less, and, for example, more than 0, preferably 0.02 or more, more preferably 0.04 or more, more preferably 1.0 or more, more preferably 2.0 or more, more preferably 3.0 or more, more preferably 4.0 or more.

The "peak area (R2) of the compound B with respect to the total peak area 100 of the compound contained in the polythiol composition" means the relative value (ratio) of the peak area ($P_B$) of the compound B when the total peak area ($P_{sum}$) of the compound contained in the polythiol composition is 100, and is calculated according to the following formula (2). The "total peak area ($P_{sum}$) of the compound contained in the polythiol composition" is the total sum of the peak area of all the peaks detected in the high-performance liquid chromatography measurement of the polythiol composition.

$$\text{Formula (2): } R2=(P_B/P_{sum})\times 100$$

When the "peak area (R2) of the compound B with respect to the total peak area 100 of the compound contained in the polythiol composition" is determined, the high performance liquid chromatography measurement is carried out under the measurement conditions described in paragraph [0049] of WO2022/138865.

When the peak area (R2) of the compound B with respect to the total peak area 100 of the compound contained in the polythiol composition is the above-described upper limit or less, it is possible to improve the light resistance of the resin produced from the polythiol composition. Further, when the peak area (R2) of the compound B with respect to the total peak area 100 of the compound contained in the polythiol composition is the above-described lower limit or more, it is possible to improve the dyeability of the resin produced from the polythiol composition.

For example, the resin is manufactured by casting molding. In the casting molding, first, the isocyanate component and the active hydrogen group-containing component are mixed in a ratio in which the isocyanate group in the isocyanate component to the active hydrogen group (amino group, thiol group, or hydroxyl group) in the active hydrogen group-containing component is 0.8 to 1.2. The resulting mixture is the polymerizable composition containing the isocyanate component and the active hydrogen group-containing component.

In addition, a known additive may be mixed into the polymerizable composition. Examples of the additive include a curing catalyst, a stabilizer (acidic phosphate ester), and an ultraviolet absorber.

Next, the polymerizable composition is injected into a mold, and then heated and cured. Thus, a molded article made of the resin is obtained. In other words, the resin is a cured product of the polymerizable composition.

When the active hydrogen group-containing component contains the polythiol, the resulting molded article is excellent in transparency.

Further, the above-described polyisocyanate composition contains XDI and a specific compound, the resulting molded article has a high refractive index and excellent heat resistance.

Specifically, the refractive index (ne) of the resulting molded article is, for example, 1.650 or more, preferably 1.660 or more, and, for example, 1.670 or less.

The Abbe number (νe) of the resulting molded article is, for example, 30 or more, preferably 31 or more, and, for example, 35 or less, preferably 33 or less.

Further, the glass-transition temperature (Tg) of the resulting molded article is, for example, 80°C or more, preferably 85°C or more, and, for example, 95°C or less.

The resulting molded article also has a low YI value. Specifically, the YI value of the resulting molded article is, for example, 2.00 or less, preferably 1.50 or less, more preferably 1.20 or less, more preferably 1.10 or less, more preferably 1.05, and, for example, 1.00 or more.

When having the above-described physical properties, the resulting molded article is suitable as an optical element.

Examples of the optical element include a lens, a sheet, and a film, and preferably a lens is used.

Examples of the lens include, for example, a transparent lens, a sunglasses lens, a polarizing lens, an eyeglass lens, a camera lens, a pickup lens, and a contact lens.

The uses of the XDI composition are not limited to the above-described optical materials. The uses of the XDI composition include inks, transfer foils, pressure-sensitive adhesives, binders, gels, elastomers, foams, adhesives, one-component curing sealants, RIM moldings, microcell polyurethanes, various microcapsules, aqueous resins, thermosetting resins, active energy rays (e.g., electron beam, ultraviolet light, etc.) curable resins, artificial and synthetic leathers, slush powders, robotic members, mobility members, health care materials, substrate resins of carbon-fiber reinforced plastics (CFRPs), transparent rubber, transparent hard resins, waterproof materials, films, sheets, tubes, blades, loudspeakers, sensors, organic EL members, photovoltaic members, android members, wearable members, sports items, leisure items, medical products, nursing care products, house components, audio components, lighting components, chandeliers, outdoor wall lights, packings, vibration-proof, vibration-control, and seismic-isolated members, acoustic insulation components, daily use items, sundry items, cushions, bedclothes, stress absorbers, stress relaxation materials, car interior or exterior materials, components for transportation, components for office automation equipment, sundry item-surface protection materials, self-repairing materials, and health appliances.

The uses of the XDI composition preferably include the above-described optical materials, elastomers, foams, and one-component curing sealants.

4. Operations and Effects

The xylylene diisocyanate composition and polymerizable composition of the present invention contain the above-described specific compound.

Therefore, by using the xylylene diisocyanate composition and the polymerizable composition as a raw material, a resin having excellent heat resistance can be produced.

The resin of the present invention is a cured product of the polymerizable composition containing the above-described xylylene diisocyanate composition.

Further, the molded article, optical element, and lens of the present invention are made of the resin.

Therefore, the resin, molded article, optical element, and lens of the present invention have excellent heat resistance.

5. Modified Examples

(1) The method of synthesis of XDI is not limited to the above-described hydrochloride method. Examples of the method of synthesis of XDI include a gas phase method in which evaporated XDA is reacted with a carbonyl dichloride, a single step method in which XDA and a carbonyl dichloride are directly reacted in one step, and a cold and hot two-step method in which XDA and a carbonyl dichloride are reacted at a low temperature and then subsequently reacted at a high temperature. In these methods, XDI is obtained by reacting XDA with a carbonyl dichloride. Further, examples of a method of synthesis of XDI include a non-phosgene method in which a xylylene dicarbamate is thermally decomposed to obtain XDI.

(2) The production of the XDI composition may not be carried out continuously in the same plant. For example, the first XDI composition may be produced in the first plant and then used to carry out the generation step in the second plant.

## Examples

Next, the present invention is described in reference to Examples below. The present invention is however not limited by the following Examples. The specific numeral values used in the description below, such as mixing ratios (content ratios), physical property values, and parameters, can be replaced with the corresponding mixing ratios (content ratios), physical property values, and parameters in the above-described "DESCRIPTION OF THE EMBODIMENTS", including the upper limit values (numeral values defined with "or less", and "less than") or the lower limit values (numeral values defined with "or more", and "more than"). The "ratio" of each component is based on mass.

### 1. Production of XDI composition

#### (1) Example 1

A XDI composition (first XDI composition) was produced by the production method described in WO 2018-190290.

50 mg of PTSA (manufactured by FUJIFILM Wako Pure Chemical Corporation) was added to 100 g of the produced XDI composition, and the mixture was stirred at 60°C for 6 hours. Thereafter, at room temperature, 10 mg of PTSI (manufactured by Tokyo Chemical Industry Co., Ltd.) was added thereto and mixed homogenously. Thus, a XDI composition (second XDI composition) was produced.

#### (2) Example 2

80 mg of PTSA (manufactured by FUJIFILM Wako Pure Chemical Corporation) was added to 100 g of the XDI composition (first XDI composition) obtained by the production method described in WO2018-190290, and the mixture was stirred at 60°C for 10 hours. Thus, a XDI composition (second XDI composition) was produced.

#### (3) Example 3

150 mg of PTSA (manufactured by FUJIFILM Wako Pure Chemical Corporation) was added to 100g of the XDI composition (first XDI composition) obtained by the production method described in WO2018-190290, and the mixture was stirred at 60°C for 10 hours. Thereafter, at room temperature, 60 mg of PTSI (manufactured by Tokyo Chemical Industry Co., Ltd.) was added and mixed homogenously. Thus, a XDI composition (second XDI composition) was produced.

#### (4) Example 4

A XDI composition (second XDI composition) produced by the same production method as Example 1 was stirred at 100°C for 2 hours. Thereafter at a room temperature, 40mg of PTSI (manufactured by Tokyo Chemical Industry Co., Ltd.) was added thereto, and mixed homogenously. Thus, a XDI composition (second XDI composition) was produced.

#### (5) Example 5

300 mg of PTSA (manufactured by FUJIFILM Wako Pure Chemical Corporation) was added to 100 g of the XDI composition (first XDI composition) obtained by the production method described in WO2018-190290, and the mixture was stirred at 80°C for 5 hours. Thereafter, at room temperature, 80 mg of PTSI (manufactured by Tokyo Chemical Industry Co., Ltd.) was added thereto, and mixed homogenously. Thus, a XDI composition (second XDI composition) was produced.

#### (6) Example 6

300 mg of PTSA (manufactured by FUJIFILM Wako Pure Chemical Corporation) was added to 100g of the XDI composition (first XDI composition) obtained by the production method described in WO2018-190290, and the mixture was stirred at 80°C for 6 hours. Thereafter, at room temperature, 100 mg of PTSI (manufactured by Tokyo Chemical Industry Co., Ltd.) was added thereto, and mixed homogenously. Thus, a XDI composition (second XDI composition) was produced.

(7) Example 7

200 mg of PTSA (manufactured by FUJIFILM Wako Pure Chemical Corporation) was added to 100 g of the XDI composition (first XDI composition) obtained by the production method described in WO2018-190290, and the mixture was stirred at 100°C for 4 hours. Thereafter, at room temperature, 50 mg of PTSI (manufactured by Tokyo Chemical Industry Co., Ltd.) was added thereto, and mixed homogenously. Thus, a XDI composition (second XDI composition) was produced.

(8) Example 8

200 mg of PTSA (manufactured by FUJIFILM Wako Pure Chemical Corporation) was added to 100 g of the XDI composition (first XDI composition) obtained by the production method described in WO2018-190290, and the mixture was stirred at 100°C for 4 hours. Thereafter, at room temperature, 300 mg of PTSA and 300 mg of PTSI (manufactured by Tokyo Chemical Industry Co., Ltd.) were added thereto, and mixed homogenously. Thus, a XDI composition (second XDI composition) was produced.

(9) Comparative Example 1

150 mg of PTSA (manufactured by FUJIFILM Wako Pure Chemical Corporation) was added to 100g of the XDI composition obtained by the production method described in WO2018-190290, and mixed homogenously at room temperature. Thus, a XDI composition was produced.

(10) Comparative Example 2

100 mg of PTSI (manufactured by Tokyo Chemical Industry Co., Ltd.) was added to 100 g of the XDI composition obtained by the production method described in WO2018-190290, and mixed homogenously at room temperature. Thus, a XDI composition was produced.

(11) Comparative Example 3

50 mg of PTSA (manufactured by FUJIFILM Wako Pure Chemical Corporation) and 50 mg of PTSI (manufactured by Tokyo Chemical Industry Co., Ltd.) were added to 100 g of the XDI composition obtained by the production method described in WO2018-190290, and mixed homogenously at room temperature. Thus, a XDI composition was produced.

2. Quantitative Measurement of XDI Composition

The ratios of XDI, PTSA, PTSI, Urea compound, DCI, and CBI in the XDI composition obtained in each of Examples and Comparative Examples were measured by the following method. The results are shown in Table 1 and Table 2.

(1) Ratios of XDI, PTSA, and PTSI in XDI Composition

100 mg of XDI composition and 100 mg of 1,2,4,5-tetrachlorobenzene as an internal standard material were mixed together. Next, the obtained mixture was diluted with dichloromethane so that the volume was 10 mL, thereby obtaining a sample. Next, the obtained sample was subjected to a gas chromatography analysis under the following measurement conditions.

(Measurement Conditions)

Equipment; SHIMADZU 2014 (manufactured by Shimadzu Corporation)
Packing material; DB-1 (film thickness) 1.5 μm,
Column; internal diameter 0.53 mm × length 60 m (manufactured by Shimadzu Corporation)
Oven temperature; rising from 130°C to 220°C at 3°C/min, after reaching 220°C, rising to 300°C at 10°C/min.

Split ratio; pulsed splitless method
Injection port temperature; 280°C
Detector temperature; 300°C
Carrier gas; $N_2$ 158 kPa, $H_2$ 55 kPa, Air 45 kPa (Constant pressure control)
Injection volume; 2 μL
Detection Method; FID

The ratio of XDI in the XDI composition was calculated from the area ratio based on the peak of the internal standard material which appeared at the retention time of 8.8 minutes and the peak of the XDI which appeared at the retention time of 13.8 minutes.
In addition, the ratio of PTSA in the XDI composition was calculated from the area ratio based on the peak of the internal standard material and the peak of PTSA which appeared at the retention time of 15.0 minutes.
In addition, the ratio of PTSI in the XDI composition was calculated from the area ratio based on the peak of the internal standard material and the peak of PTSI which appeared at the retention time of 9.1 minutes.

(2) Ratio of Specific Compound in XDI Composition

The infrared spectrum of the XDI composition was measured with an FTIR spectrophotometer (trade name: IR Spirit, manufactured by Shimadzu Corporation).
In the infrared spectrum of the XDI composition in Examples 1-8, the peak considered to be derived from the urea bond was observed from 1500 $cm^{-1}$ to 1650 $cm^{-1}$. On the other hand, in the infrared spectrum of the XDI composition in Comparative Examples 1-3, no peak considered to be derived from the urea bond was observed from 1500 $cm^{-1}$ to 1650 $cm^{-1}$.
$^1$H-NMR of the XDI composition was also measured.
In detail, 35 mg of the XDI composition, 0.7 mL of deuterated chloroform, and 25 mg of paraxylene (manufactured by FUJIFILM Wako Pure Chemical Corporation) as an internal standard material were mixed to obtain a sample for measurement.
The obtained sample was subjected to $^1$H-NMR measurement by setting the observation nucleus to H (400MHz), the observation range to 15 ppm, and the accumulation number to 64 times using a nuclear magnetic resonance device (ECX-400P, manufactured by JEOL Ltd.).
In the $^1$H-NMR spectrum of the XDI composition in Examples 1 to 8, a plurality of peaks was observed between the chemical shift values 2.44 and 2.46 ppm based on chloroform in deuterated chloroform. On the other hand, in the $^1$H-NMR spectrum of the XDI composition in Comparative Examples 1 to 3, no peak was observed between the chemical shift values 2.44 and 2.46 ppm.
From the comparison between the infrared spectrum and the $^1$H-NMR spectrum, it is considered that a plurality of peaks between the chemical shift values 2.44 and 2.46 ppm indicate the urea compound represented by the above-described chemical formula (1), the urea compound represented by the above-described chemical formula (2), and a derivative obtained from XDI and PTSI.
Note that the peak of the internal standard substance was observed at the chemical shift value 2.30 ppm.
Next, the ratio of the urea compound in the XDI composition in a case in which a plurality of peaks between the chemical shift values 2.44 and 2.46 ppm was at least one of the urea compound represented by the above-described chemical formula (1) and the urea compound represented by the above-described chemical formula (2) was calculated.
In detail, the mass ratio of the urea compound to the internal standard substance was calculated from the total sum of the peak areas of the plurality of peaks between the chemical shift values 2.44 and 2.46 ppm and the peak area of the peak of the internal standard substance.
The ratio of the urea compound in the XDI composition was calculated from the obtained mass ratio and the ratio of the internal standard substance in the sample.

(5) Ratio of DCI in XDI Composition

The ratio of DCI in the XDI composition was measured by the method described in paragraphs [0375] and [0376] of WO2018/190290.

(6) Ratio of CBI in XDI Composition

The ratio of CBI in the XDI composition was measured by the method described in paragraphs [0376] and [0377] of WO2018/190290.
[Table 1]

Table 1

| | | Ex. 1 | Ex. 2 | Ex. 3 | Ex. 4 | Ex. 5 | Ex. 6 | Ex. 7 | Ex. 8 |
|---|---|---|---|---|---|---|---|---|---|
| Content ratio | XDI (%) | 99.8 | 99.8 | 99.6 | 99.7 | 99.6 | 99.6 | 99.5 | 98.9 |
| | PTSA (ppm) | 400 | 500 | 900 | 0 | 1100 | 1000 | 0 | 3000 |
| | PTSI (ppm) | 100 | 0 | 600 | 400 | 800 | 1000 | 500 | 3000 |
| | Urea compound (ppm) | 300 | 500 | 800 | 1200 | 2700 | 3000 | 3200 | 4000 |
| | CBI (ppm) | 1000 | 1000 | 1000 | 1000 | 1000 | 1000 | 1000 | 1000 |
| | DCI (ppm) | 1 | 1 | 1 | 1 | 1 | 1 | 1 | 1 |
| Resin properties | YI | 1.02 | 1.05 | 1.08 | 1.09 | 1.10 | 1.15 | 1.30 | 1.35 |
| | Tg (°C) | 90.1 | 90.1 | 90.2 | 90.6 | 90.7 | 91.1 | 91.2 | 90.8 |

[Table 2]

Table 2

| | | Comp. Ex. 1 | Comp. Ex. 2 | Comp. Ex. 3 |
|---|---|---|---|---|
| Content ratio | XDI (%) | 99.8 | 99.8 | 99.8 |
| | PTSA (ppm) | 1500 | 0 | 500 |
| | PTSI (ppm) | 0 | 1000 | 500 |
| | Urea compound (ppm) | 0 | 0 | 0 |
| | CBI (ppm) | 1000 | 1000 | 1000 |
| | DCI (ppm) | 1 | 1 | 1 |
| Resin properties | YI | 1.00 | 0.99 | 0.97 |
| | Tg (°C) | 88.9 | 87.4 | 87.1 |

2. Production of Molded Article

0.01 parts by mass of dimethyltin dichloride as a curing catalyst, 0.10 parts by mass of ZELEC UN (trade name manufactured by Stepan Company; acidic phosphate ester), and 1.5 parts by mass of Biosorb 583 (manufactured by SAKAI CHEMICAL INDUSTRY CO.,LTD.; ultraviolet absorber) were mixed into 50.8 parts by mass of the XDI composition shown in Table 1 at 20°C, and dissolved to obtain a mixed solution 1.

Next, 49.2 parts by mass of a polythiol composition containing 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane as primary components was homogenously mixed into the mixed solution 1 to obtain a mixed solution 2 (polymerizable composition).

The mixed solution 2 was defoamed at 600Pa for 1 hour, and then filtered through a 1μm Teflon (registered trademark) filter.

Next, the filtered mixed solution 2 was injected into a mold formed of a glass mold and a tape.

Next, the mold into which the mixed solution 2 was injected was put into an oven, and the temperature was raised from 25°C to 120°C, and the mixed solution 2 was cured at 120°C for 24 hours.

Thereafter, the mold was removed from the oven, and the cured product was released from the mold, and the obtained cured product was annealed at 120°C for 1 hour.

As above-described, the cured product (molded article) of each of Examples and Comparative Examples was obtained.

3. Evaluations of Physical Properties of Molded Articles

(1) Refractive index (ne) and Abbe number (νe)

A test piece of length 10mm, width 10mm, and thickness 2.5mm was produced from the obtained resin, and the

refractive index (ne) at a wave length 546.1 nm (mercury e-line), the refractive index (nF') at a wave length 480.0nm (Cd F' line), and the refractive index (nC') at a wave length 643.9 nm (Cd C' line) were each measured. The Abbe number (νe) was obtained based on the refractive index (ne), the refractive index (nF'), and the refractive index (nC').

In all of Examples and Comparative Examples, the refractive index (ne) was 1.665 and the Abbe number (νe) was 31,

(2) YI Value

A circular flat plate with a diameter 75mm and a thickness 2.5mm was produced from the obtained resins, and the YI value was obtained using a spectrophotometric colorimeter CM-5 manufactured by KONICA MINOLTA JAPAN, INC.

The smaller the YI value is, the lower the yellowness of the resin is, and the larger the YI value is, the higher the yellowness of the resin is.

The YI value of each Example is shown in Table 1, and the YI value of each Comparative Example is shown in Table 2.

(3) Heat Resistance

A test piece of length 10mm, width 10mm, and thickness 2.5mm was produced from the obtained resin, and the glass-transition temperature (Tg) was measured by a TMA penetration method (50g load, pin end 0.5 mmφ, temperature increase rate 10°C/min) using a thermomechanical analyzer TMA-60 manufactured by Shimadzu Corporation. The higher the glass-transition temperature (Tg) is, the better the heat resistance is.

The glass-transition temperature (Tg) of each Example is shown in Table 1, and the glass-transition temperature (Tg) of each Comparative Example is shown in Table 2.

While the illustrative embodiments of the present invention are provided in the above-described description, such is for illustrative purpose only and it is not to be construed as limiting in any manner. Modification and variation of the present invention that will be obvious to those skilled in the art is to be covered by the following claims.

Industrial Applicability

The xylylene diisocyanate composition, polymerizable composition, resin, and molded article of the present invention can be used, for example, for producing an optical element such as a lens.

## Claims

**1.** A xylylene diisocyanate composition comprising:

a xylylene diisocyanate; and
a specific compound having a peak in a range of chemical shift values of 2.44 ppm to 2.46 ppm based on chloroform in deuterated chloroform when the xylylene diisocyanate composition is mixed with the deuterated chloroform and measured by $^1$H-NMR measurement.

**2.** The xylylene diisocyanate composition according to claim 1, wherein a ratio of the specific compound is 100 ppm or more based on mass.

**3.** The xylylene diisocyanate composition according to claim 1, wherein a ratio of the specific compound is 10000 ppm or less based on mass.

**4.** The xylylene diisocyanate composition according to claim 1, wherein a ratio of the specific compound is 3000 ppm or less based on mass.

**5.** The xylylene diisocyanate composition according to claim 1, comprising:
at least one of a urea compound represented by the following chemical formula (1) and a urea compound represented by the following chemical formula (2), as the specific compound.

Chemical formula (1):

[Chemical formula 1]

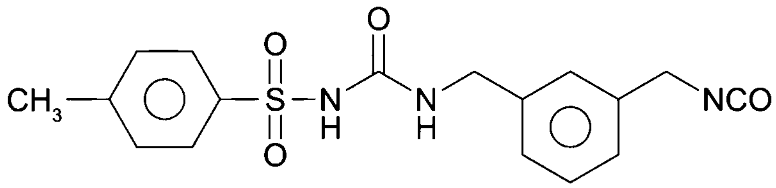

(1)

Chemical formula (2):

[Chemical formula 2]

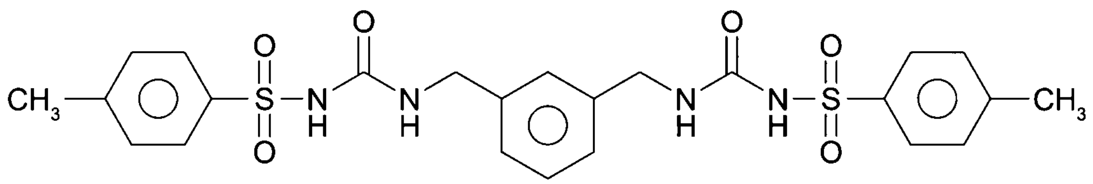

(2)

**6.** The xylylene diisocyanate composition according to claim 1, further comprising:
at least one of 4-methylbenzenesulfonamide and 4-methylbenzenesulfonyl isocyanate.

**7.** The xylylene diisocyanate composition according to claim 6, wherein a total sum of a ratio of the 4-methylbenzenesulfonamide in the xylylene diisocyanate composition and a ratio of the 4-methylbenzenesulfonyl isocyanate in the xylylene diisocyanate composition is 2000 ppm or less based on mass.

**8.** The xylylene diisocyanate composition according to claim 1, further comprising:
at least one of monochloromethylbenzyl isocyanate and dichloromethylbenzyl isocyanate.

**9.** The xylylene diisocyanate composition according to claim 1, wherein the xylylene diisocyanate is obtained by reaction of xylylenediamine with carbonyl dichloride or by reaction of a hydrochloride salt of xylylenediamine with carbonyl dichloride.

**10.** A polymerizable composition comprising:

the xylylene diisocyanate composition according to any one of claims 1 to 9; and
an active hydrogen group-containing component.

**11.** The polymerizable composition according to claim 10, wherein the active hydrogen-containing component contains at least one type of polythiol selected from the group consisting of 5,7-dimercaptomethyl-1,11-dimercapto-3,6,9-trithiaundecan, 4,7-dimercaptomethyl-1,11-dimercapto-3,6,9-trithiaundecan, 4,8-dimercaptomethyl-1,11-dimercapto-3,6,9-trithiaundecan, 4-mercaptomethyl-1,8-dimercapto-3,6-dithiaoctane, pentaerythritol tetrakis(2-mercaptoacetate), pentaerythritol tetrakis(3-mercaptopropionate), 2,5-bis(mercaptomethyl)-1,4-dithiane, bis(mercaptoethyl) sulfide, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane, 1,1,2,2-tetrakis(mercaptomethylthio)ethane, 3-mercaptomethyl-1,5-dimercapto-2,4-dithiapentane, tris(mercaptomethylthio)methane, and ethylene glycol bis(3-mercaptopropionate).

**12.** A resin being a cured product of the polymerizable composition according to claim 11.

**13.** A molded article made of the resin according to claim 12.

**14.** An optical element being the molded article according to claim 13.

**15.** A lens being the optical element according to claim 14.

## INTERNATIONAL SEARCH REPORT

International application No.
**PCT/JP2024/029210**

### A. CLASSIFICATION OF SUBJECT MATTER

***C08G 18/76***(2006.01)i; ***C07C 265/14***(2006.01)i; ***C08G 18/38***(2006.01)i; ***G02B 1/04***(2006.01)i; ***G02C 7/00***(2006.01)i
FI: C08G18/76 014; C08G18/38; C07C265/14; G02B1/04; G02C7/00

According to International Patent Classification (IPC) or to both national classification and IPC

### B. FIELDS SEARCHED

Minimum documentation searched (classification system followed by classification symbols)

C08G18/76; C07C265/14; C08G18/38; G02B1/04; G02C7/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | WO 2019/009230 A1 (MITSUI CHEMICALS, INC.) 10 January 2019 (2019-01-10) claims, comparative example 5, table 1 | 1-15 |
| A | WO 2023/037645 A1 (MITSUI CHEMICALS, INC.) 16 March 2023 (2023-03-16) claims, paragraphs [0036]-[0037], examples | 1-15 |
| A | JP 2019-59821 A (MITSUI CHEMICALS, INC.) 18 April 2019 (2019-04-18) claims, example 1 | 1-15 |
| A | JP 9-87239 A (MITSUBISHI GAS CHEMICAL COMPANY, INC.) 31 March 1997 (1997-03-31) claims, example 2 | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 October 2024** | **05 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**
**Information on patent family members**

International application No.
**PCT/JP2024/029210**

| Patent document cited in search report | Publication date (day/month/year) | Patent family member(s) | Publication date (day/month/year) |
|---|---|---|---|
| WO 2019/009230 A1 | 10 January 2019 | US 2020/0148856 A1<br>claims, comparative example 5, table 1<br>EP 3650480 A1<br>CN 110831992 A | |
| WO 2023/037645 A1 | 16 March 2023 | EP 4198069 A1<br>claims, paragraphs [0037]-[0038], examples<br>KR 10-2023-0038786 A<br>CN 116018366 A | |
| JP 2019-59821 A | 18 April 2019 | (Family: none) | |
| JP 9-87239 A | 31 March 1997 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018190290 A
- WO 2022102625 A
- WO 2022138865 A